# EUROPÄISCHE PATENTSCHRIFT

(11) **EP 3 460 452 B1**
(45) Veröffentlichungstag und Bekanntmachung des Hinweises auf die Patenterteilung: **04.09.2019**
(21) Anmeldenummer: 17192412.9
(22) Anmeldetag: 21.09.2017
(51) Int. Cl.: G01N 21/15, G01N 33/00, G01N 21/53, G01N 21/85, G01N 1/22, G01N 21/3504

(54) **GASANALYSATOR ZUR OPTISCHEN GASANALYSE**
GAS ANALYSER FOR OPTICAL GAS ANALYSIS
ANALYSEUR DE GAZ DESTINÉ À L'ANALYSE OPTIQUE DE GAZ

(43) Veröffentlichungstag der Anmeldung: 27.03.2019
(73) Patentinhaber: Sick AG, 79183 Waldkirch (DE)
(72) Erfinder: ZORBACH, Ralf, 79183 Waldkirch (DE)

(56) Entgegenhaltungen:
- EP-A1- 1 944 598
- EP-A2- 2 216 638
- DE-U1-202016 101 286
- US-A- 3 559 491
- US-A- 4 481 833
- US-A1- 2012 033 219
- US-A1- 2017 122 877

## Beschreibung

Die Erfindung betrifft einen Gasanalysator zur optischen In-situ Gasanalyse in einem Gasstrom, der neben einem zu messenden Gasanteil, dessen Konzentration bestimmt werden soll, die Konzentrationsbestimmung störende Partikel, wie Staub, Rauch, Wasser oder sonstige Aerosole, enthält, mit einem Lichtsender und einem Lichtempfänger, die zusammen eine optische Messtrecke in einem Messvolumen definieren, einer Auswerteeinrichtung zur Auswertung der Empfangssignale des Lichtempfängers zur Konzentrationsbestimmung und einer Ablenkvorrichtung zur Ablenkung der störenden Partikel, um diese Partikel am Messvolumen vorbei strömen zu lassen.

Bei diesen Vorrichtungen handelt es sich beispielsweise um optische Spektrometer, Sichtweitenmessgeräte, In-situ Gasanalysatoren, Tunnelsensoren und dergleichen. In solchen In-situ-Gasanalysatoren werden bestimmte Gasanteile, z. B. Schwefelwasserstoff, Kohlenmonoxid, SO2, NH3, NO NO2, HCl, HF oder dergleichen, aus dem Spektrum bzw. der Absorption, verursacht durch die Gasmatrix, ermittelt.

Anwendungsgebiete sind zum Beispiel Emissionsmessungen von Industrieanlagen, bei denen die Abgase auf ihren Gehalt bestimmter molekularer Verbindungen überwacht werden müssen. Häufig sind die Gasströme, denen die optoelektronische Vorrichtung ausgesetzt ist um die gewünschten Gasanteile zu messen, durch hohe Partikelbelastungen, wie zum Beispiel Rauch, Staub, Wassertröpfchen oder andere Aerosole, gekennzeichnet. Diese hohen Partikelbelastungen verursachen eine große Lichtabsorption und/oder eine hohe Lichtstreuung, die die eigentliche Messung stark behindert bis unmöglich macht. So hat beispielsweise Schwefelwasserstoff eine sehr breite Absorption genauso wie zum Beispiel auch ultrafeiner Staub. Es kann dann nicht mehr unterschieden werden, ob die Absorption vom Schwefelwasserstoff herrührt oder von dem Staub. Die störenden Partikel in der Gasmatrix vergrößern somit die Absorption und verkleinern damit den Signal-RauschAbstand. Das zulässige Maximum der Absorption bzw. der minimale Signal-RauschAbstand wird durch Messtechnik und Auswerteverfahren bestimmt. Überschreitungen des Maximums führen zu Fehlermeldungen, Messfehlern oder hohem Messwertrauschen. Die Verfügbarkeit wird eingeschränkt oder eine Messung unmöglich. Eine Reduzierung der aktiven gasdurchströmten Messstrecke würde zwar die störenden Absorptionen verringern, jedoch parallel dazu auch die Empfindlichkeit der Gasmessung verringern.

Zur Reduzierung der störenden Absorptionen durch die störenden Partikel ist es aus der EP 2 405 254 B1 bekannt, einen Elektrofilter einzusetzen, der die Partikel ionisieren, ablenken und an der Messstrecke vorbeiführen soll. Ein solcher Elektrofilter ist relativ aufwändig, bedarf einer Energieversorgung und ist darüber hinaus nicht ausreichend effektiv.

Die US 2012/0033219 A1 offenbart einen Gasanalysator, der zur Reduktion der Konzentration von störenden Partikeln in der Messstrecke eine Ablenkvorrichtung mit Öffnungen auf der strömungsabgewandten Seite verwendet. Die DE 20 2016 101 286 U1 beschreibt einen Gasanalysator, der eine Reduktion der Konzentration störender Partikel in der Messstrecke mittels eines Filters erzielt.

Ausgehend von diesem Stand der Technik ist es Aufgabe der Erfindung, einen verbesserten Gasanalysator zur optischen Gasanalyse bereitzustellen, mit der der störende Einfluss der im Medium enthaltenen Partikel auf die beabsichtigte Messung noch besser verringert ist.

Diese Aufgabe wird gelöst durch einen Gasanalysator mit den Merkmalen des Anspruchs 1.

Ein erfindungsgemäßer Gasanalysator zur optischen In-situ Gasanalyse in einem Gasstrom, der neben einem zu messenden Gasanteil, dessen Konzentration bestimmt werden soll, die Konzentrationsbestimmung störende Partikel, wie Staub, Rauch, Wassertröpfchen oder sonstige Aerosole, enthält, umfasst einen Lichtsender und einen Lichtempfänger, die zusammen eine optische Messtrecke in einem Messvolumen definieren, eine Auswerteeinrichtung zur Auswertung von Empfangssignalen des Lichtempfängers zur Konzentrationsbestimmung und eine Ablenkvorrichtung zur Ablenkung der störenden Partikel, um diese Partikel am Messvolumen vorbei strömen zu lassen. Erfindungsgemäß ist die Ablenkvorrichtung als quer zur Gasströmung liegendes Mantelrohr ausgebildet, in dessen Innerem das Messvolumen liegt, wobei das Mantelrohr einen geschlossenen Mantel aufweist und die Stirnseiten Öffnungen zum Durchlassen von Gas und Licht des Lichtsenders in das Messvolumen aufweisen.

Die Erfindung macht sich im Kern zu Nutze, dass die störenden Partikel, also die Aerosole wie Wassertröpfchen, Staub, Rauchteilchen, schwerer und damit träger sind als die zu messenden Gasanteile aus dem Messgas. Durch das quer zur Strömung liegende, umfangsmäßig geschlossene aber endseitig offene Mantelrohr finden die trägeren, schwereren Partikel nicht oder zumindest schwerer den Weg in das Messvolumen, denn dafür müssen die Partikel aus der Strömung abgelenkt werden und durch die endseitigen Öffnungen des Mantelrohres ins Innere gelangen. Da die leichteren Gasanteile diesen Weg besser finden ist der Anteil störender Partikel in dem Messvolumen, also auf der Messstrecke, reduziert. Die zu messenden, wesentlich leichteren Gasanteile verteilen sich in unveränderter Konzentration im Messvolumen. Weiter kommt es durch das Mantelrohr, das gegen die Strömung schützt, allein durch die Gravitation zu einer Tropfen-Aerosol-Staub-Abscheidung aus dem Gas. Der Gasaustausch zwischen Messvolumen und außerhalb des Messvolumens erfolgt 'nur' durch die endseitigen Öffnungen aufgrund von Differenzdrücken und Diffusion. Die Trennung ist sehr effektiv und verursacht wenig Aufwand.

Durch die Erfindung werden bisher nicht mögliche Gasanalysen unter extremen Anlagenbedingungen mit höchsten Partikelbelastungen möglich.

Für die Gasanalyse müssen keine Proben entnommen werden. Jegliche Aufbereitung solcher Proben entfällt.

Es wird keine Energie verbraucht, kein sonstiges Material, z.B. Filtermaterial, verbraucht, woraus sich keinerlei Erhöhung der Betriebskosten ergeben.

Die Absorptions-/Extinktionsreserve für die Gasmessung wird erhöht, indem der Signal-Rauschabstand und die Messwertstabilität vergrößert sind und indirekt die Messgenauigkeit erhöht ist. Es ist keine Messstreckenverkürzung auf Grund von Tröpfchen, Staub und Aerosolen auf Kosten der Messgenauigkeit erforderlich.

Die Erfindung ist bei sogenannten Cross-Duct wie auch Lanzen-Version einsetzbar. Die Wartung und Instandhaltung sind denkbar einfach. Eine Reinigung (wenn erforderlich) kann deshalb auch durch ungeschultes Personal erfolgen. Bestehende Anlagen/Analysatoren können nachgerüstet werden.

Vorteilhafterweise sind an den Stirnseiten des Mantelrohres Leitbleche vorgesehen, um die Gasströmung so führen zu können, dass der Trenneffekt noch erhöht ist. In einer ersten Ausführung sind die Leitbleche flach ausgebildet und haben vorzugsweise die Form eines Deckels für die Stirnseiten.

Es ist vorteilhaft, wenn in einer Ausführungsform die Öffnungen in den Stirnseiten kleiner sind als der Querschnitt des Mantelrohres im Bereich des Messvolumens. Denn dann kommt es im Messvolumen zu einer Verlangsamung der Strömung und damit Strömungsberuhigung. Das hat den Effekt, dass sich die störenden Partikel, die trotz der Umlenkung in das Mantelrohr gelangen, leichter absetzen können und dadurch von den leichteren Gasanteilen abgeschieden werden und sich nicht mehr in der Messstrecke befinden.

Die Abtrennung der störenden Partikel kann noch effektiver erfolgen, wenn jede der Öffnungen der Stirnseiten einen Ablenkring aufweist. Wenn die schweren Partikel an einen solchen Ablenkring prallen, erfahren sie eine Ablenkung in eine andere Richtung. Die Ablenkringe sind so ausgestaltet, dass diese Ablenkung von der Öffnung weg gerichtet ist. Das kann zum Beispiel durch eine kegelabschnittsförmige Form der Stirnseite erfolgen, so dass die Stirnseite des Mantelrohres leicht spitz zuläuft mit der Öffnung auf der Spitze. Dadurch wird auch ein "Einschälen" der schweren Partikel in das Rohr wirksam verhindert.

Schließlich kann in Weiterbildung der Erfindung eine Spülluftvorrichtung vorgesehen sein, mit der ein Druckluftstoss durch das Mantelrohr blasbar ist, um das Mantelrohr von Zeit zu Zeit (z.B. im 2-Stundentakt) von sich darin abgelagerten Partikeln zu befreien. Dies ist insbesondere in extremen Hoch-Staub-Applikationen sinnvoll. Durch einen oder mehrere Druckluftstösse können die Partikel aufgewirbelt und ausgeblasen werden.

Im Folgenden wird die Erfindung anhand von Ausführungsbeispielen unter Bezugnahme auf die Zeichnung im Einzelnen erläutert. In der Zeichnung zeigen:
- Fig. 1: eine schematische Darstellung einer ersten Ausführungsform eines erfindungsgemäßen Gasanalysators in einem Gasstrom;
- Fig. 2: die Vorrichtung aus Fig. 1 in einer Schnittdarstellung entlang der Linie II-II aus Fig. 1;
- Fig. 3: eine schematische Darstellung einer zweiten Ausführungsform;
- Fig. 4: eine schematische Darstellung einer dritten Ausführungsform;
- Fig. 5: eine schematische Darstellung einer vierten Ausführungsform.

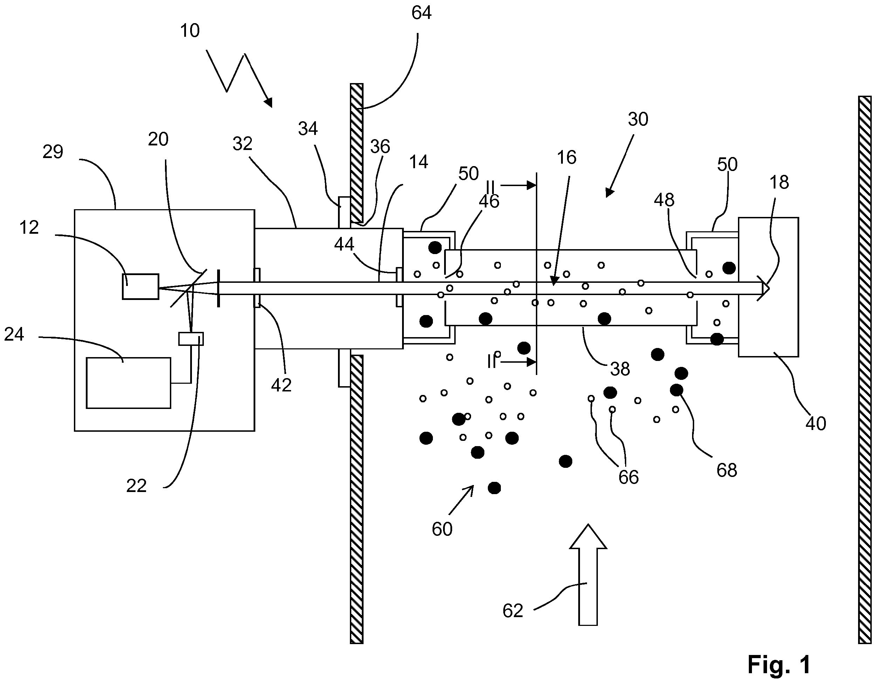

Ein erfindungsgemäßer Gasanalysator 10 zur Gasanalyse eines Gasstroms 60, der in Richtung 62 in einem Kanal 64 strömt, weist in einem in Fig. 1 dargestellten, ersten Ausführungsbeispiel einen Lichtsender 12 auf, der einen Sendelichtstrahl 14 aussendet. Der Sendelichtstrahl 14 definiert ein Messvolumen 16 und wird nach Reflexion an einem Retroreflektor 18 und einem Teilerspiegel 20 von einem Lichtempfänger 22 empfangen. Die durch die Lichtstrahlen 14 gebildete optische Messstrecke umfasst das Messvolumen 16.

Der Lichtempfänger 22 erzeugt in Abhängigkeit des auftreffenden Lichts Empfangssignale, die in einer Auswerteeinrichtung 24 ausgewertet werden.

Ein solcher Gasanalysator 10 kann beispielsweise als Transmissiometer ausgebildet sein, so dass mit dem Lichtempfänger 22 die Intensität des durch das Messvolumen 16 hindurchtretenden Lichts gemessen wird. In der Regel ist der Lichtsender 12 auf eine bestimmte Wellenlänge abgestimmt, die von einem zu untersuchenden Gasanteil, beispielsweise Schwefelwasserstoff, absorbiert wird. Über das am Lichtempfänger 22 empfangene Licht kann dann eine Aussage gemacht werden, wie hoch die Konzentration des interessierenden Gasanteils, z. B. von Schwefelwasserstoff, in dem Gasstrom 60 ist, der in dem Kamin 64 geführt ist.

Der Gasanalysator 10 umfasst weiter ein Gehäuse 29, mit einem lanzenartigen Fortsatz 30, wobei in dem Gehäuse 29 die optoelektronischen Einheiten, wie Lichtsender 12, Lichtempfänger 22 und Auswerteeinrichtung 24 angeordnet sind und in dem lanzenartigen Fortsatz 30 das Licht durch das Messvolumen 16 geführt ist und am Ende dieses Fortsatzes 30 der Retroreflektor 18 gehalten ist.

Der lanzenartige Fortsatz 30 ist mehrteilig aufgebaut. Direkt an das Gehäuse 29 anschließend ist ein erster Halteteil 32 angeordnet, über den der gesamte Gasanalysator mittels eines Flansches 34 in einer Öffnung 36 des Kamins 64 in der Wandung des Kamins 64 gehalten ist. An das Halteteil 32 schließt sich ein Mantelrohr 38 an, das den Kern der Erfindung bildet und dessen Funktion und Ausgestaltung weiter unten näher erläutert wird. An das Mantelrohr 38 wiederum schließt sich ein Endgehäuse 40 an, in dem der Retroreflektor 18 gehalten ist.

Alle Komponenten des lanzenartigen Fortsatzes 30 werden von dem Licht 14 in Längsrichtung des Mantelrohres 38 durchsetzt. Dazu weist das Halteteil 32 beispielsweise Fenster 42 und 44 auf. Das Mantelrohr 38 selbst weist an seinen Stirnseiten Öffnungen 46 und 48 auf, durch die das Licht 14 aber auch das Gas 60 treten kann. Halteteil 32, Mantelrohr 38 und Endgehäuse 40 sind über Haltearme 50 miteinander verbunden und gehalten. Die Haltearme 50 halten die jeweiligen Komponenten, also Halteteil 32 und Mantelrohr 38 bzw. Mantelrohr 38 und Endgehäuse 40 auf Abstand, so dass zwischen den Komponenten Platz verbleibt, durch den das Gas 60 hindurchströmen kann.

Der in dem Kamin 64 geführte Gasstrom 60 besteht aus bestimmten Gasanteilen, von denen wenigstens einer mit dem Gasanalysator 10 gemessen werden soll, also dessen Konzentration bestimmt werden soll. Ein solcher Gasanteil kann wie eingangs bereits erläutert beispielsweise Schwefelwasserstoff, Kohlenmonoxid, SO2, NH3, NO NO2, HCl, HF oder dergleichen sein. Diese zu messenden Gasanteile sind in der Zeichnung mit kleinen hohlen Kreisen 66 verdeutlicht. Weiter enthält der Gasstrom die eigentliche Gasmessung störende Partikel, bei denen es sich um Wassertröpfchen, Staub, Rauch oder sonstige Aerosole handeln kann. Diese Partikel sind in der Zeichnung mit gefüllten größeren Kreisen 68 angedeutet. Diese störenden Partikel 68 stören die eigentliche optische Messung, wenn sie sich in dem Lichtstrahl 14 befinden. Diese Partikel sind als Aerosole deutlich größer und schwerer als die zu messenden Gasanteile, die molekular sind.

Ziel der Erfindung ist es, diese Partikel 68 von dem Messvolumen 16 fern zu halten. Dazu dient das Mantelrohr 38, in dem sich der Großteil der optischen Messstrecke befindet. Lediglich die Zwischenräume zwischen Halteteil 32 und Mantelrohr 38 bzw. zwischen Mantelrohr 38 und Endgehäuse 40, die selbstverständlich auch von dem Lichtstrahl 14 durchsetzt werden, liegen außerhalb des Mantelrohres 38. Der Mantel des Mantelrohres 38 ist vollständig geschlossen, so dass kein Gas 60 hindurchtreten kann. In das Innere des Mantelrohres 38 und damit in das Messvolumen 16 kann Gas nur durch die endseitigen Öffnungen 46 und 48 gelangen. Das Gas, das also in das Messvolumen gelangt, muss eine aus der Strömungsrichtung 62, die an den Öffnungen 46 und 48 vorbeiführt, herausführende Ablenkung erfahren. Diese Umlenkung in das Messvolumen 16 erfolgt einzig aufgrund von Diffusion und Druckunterschieden. Die leichteren Gasanteile 66 sind deshalb wesentlich leichter in das Messvolumen abzulenken als die schwereren Partikel 68. Deshalb werden die Partikel 68 zu einem größeren Prozentsatz an den Öffnungen 46 und 48 vorbeiströmen als die leichteren Gasanteile 66. Das bedeutet, dass in dem Mantelrohr 38 und damit im Messvolumen 16 der Anteil an störenden Partikeln 68 niedriger ist als außerhalb des Mantelrohres. Die Gasmessung wird daher weniger gestört.

Ein weiterer günstiger Effekt des erfindungsgemäßen Gasanalysators 10 ist die Tatsache, dass in dem Mantelrohr 38 eine Strömungsberuhigung stattfindet. Dadurch wird bewirkt, dass die schwereren Partikel 68 sich im unteren Bereich des Mantelrohres 38 absetzen können und so eine weitere Erniedrigung ihres Anteils im Messvolumen 16, das in der Mitte des Mantelrohres 38 verläuft, bewirken. Das Mantelrohr 38 ist im Durchmesser deshalb bevorzugt deutlich größer als der Durchmesser des Lichtstrahls 14 bzw. Messvolumens 16 und selbstverständlich ist das Mantelrohr 38 im wesentlichen horizontal angeordnet.

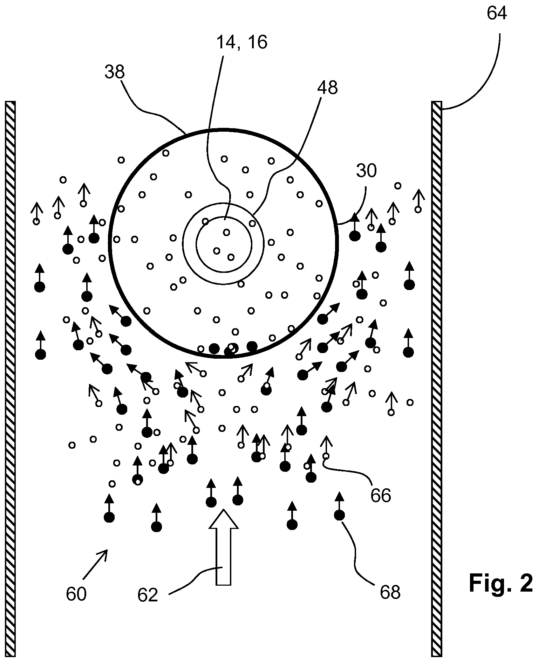

Der mehrfache Effekt des Mantelrohres 38, also die Abhaltung von störenden Partikeln 68 aus dem Inneren des Mantelrohres 38 und damit aus dem Messvolumen 16 und die Strömungsberuhigung innerhalb des Mantelrohres 38 soll nochmal in Fig. 2 verdeutlicht sein. Dort sind die Partikel 68 und die Gasanteile 66 zumindest teilweise mit kleinen Pfeilen versehen, die die Geschwindigkeit von Partikel bzw. Gasanteil andeuten soll. Das Gas mit Partikeln 68 und Gasanteilen 66 wird einerseits an dem umfangsmäßig geschlossenen Mantelrohr 38 vorbeigeführt. Nur durch die stirnseitigen Öffnungen 46 und 48 können diese in das Innere gelangen. Dort liegt aber keine Strömung vor und die Partikel 68 können sich absetzen.

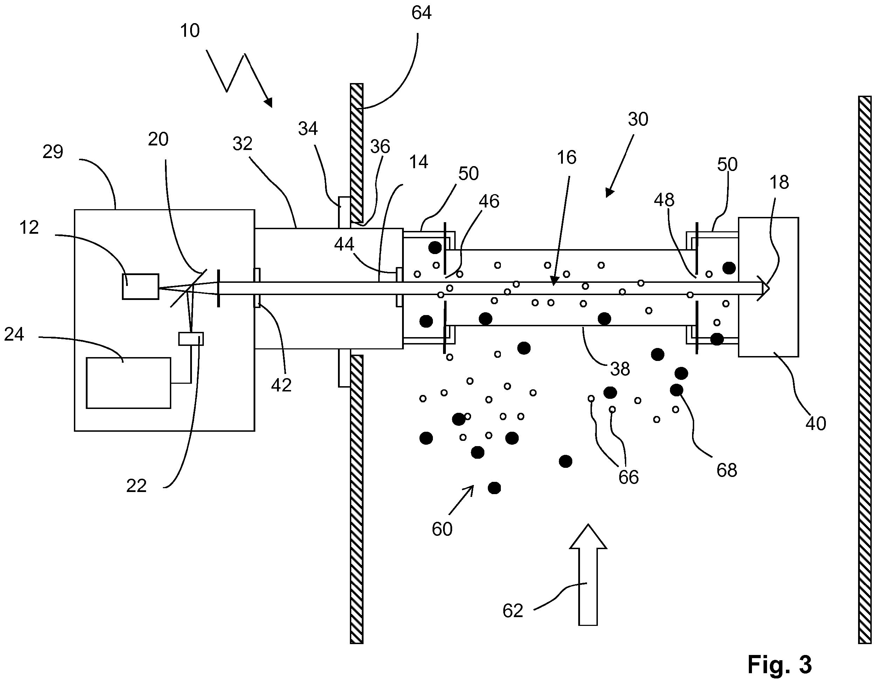

Fig. 3 zeigt ein zweites Ausführungsbeispiel, bei dem in Ergänzung zum ersten Ausführungsbeispiel Leitbleche an den Stirnseiten des Mantelrohres 38 angeordnet sind. Die Leitbleche sind jeweils quasi als flacher Deckel der Stirnseiten ausgebildet und weisen die Öffnungen 46 und 48 auf und stehen über den Durchmesser des Mantelrohres 38 hinaus. Durch die Leitbleche kann die Gasströmung besser geführt werden, so dass das Gas 60 sauber an den Öffnungen 46 und 48 vorbeiströmt und damit insbesondere die schwereren Partikel 68 an den Öffnungen 46 und 48 vorbeiströmen und nicht hineingelangen.

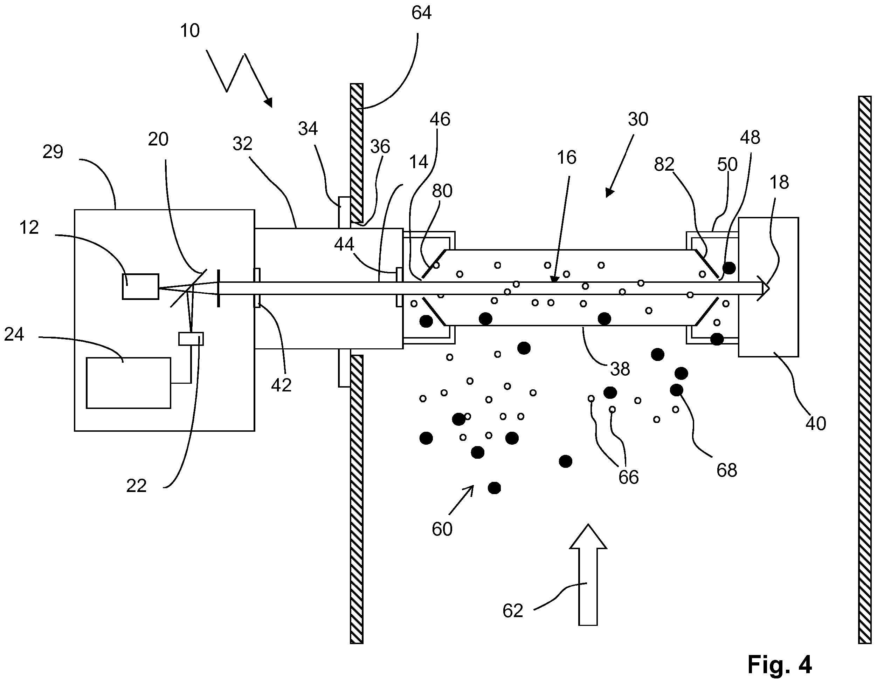

Die Trennung der schwereren Partikel 68 und der leichteren Gasanteile 66 kann weiter verbessert werden, wenn das Mantelrohr 38 an seinen Stirnseiten, an denen sich die Öffnungen 46 und 48 befinden, Ablenkringe 80 und 82 aufweist, wie dies in Fig. 4 dargestellt ist, die nur Teile des Gasanalysators 10 zeigt. Wenn die schweren Partikel 68 an einen solchen Ablenkring 80, 82 prallen, erfahren sie eine Ablenkung in eine andere Richtung. Die Ablenkringe 80 und 82 sind so ausgestaltet, dass diese Ablenkung von der Öffnung 46 bzw. 48 weg gerichtet ist. Das kann zum Beispiel durch eine kegelabschnittsförmige Form der Stirnseite des Mantelrohres 38 erfolgen, so dass die Stirnseite leicht spitz zuläuft mit der Öffnung 46 bzw. 48 an der Spitze. Prinzipiell erfahren natürlich auch die leichteren Gasanteile 66 eine solche Ablenkung, aber aufgrund ihrer geringeren Masse hat das weniger Auswirkung auf das Hineindiffundieren in das Mantelrohrinnere durch die Öffnung 46 bzw. 48 hindurch. Insgesamt wird dadurch ein "Einschälen" der schweren Partikel 68 in das Rohr wirksam verhindert.

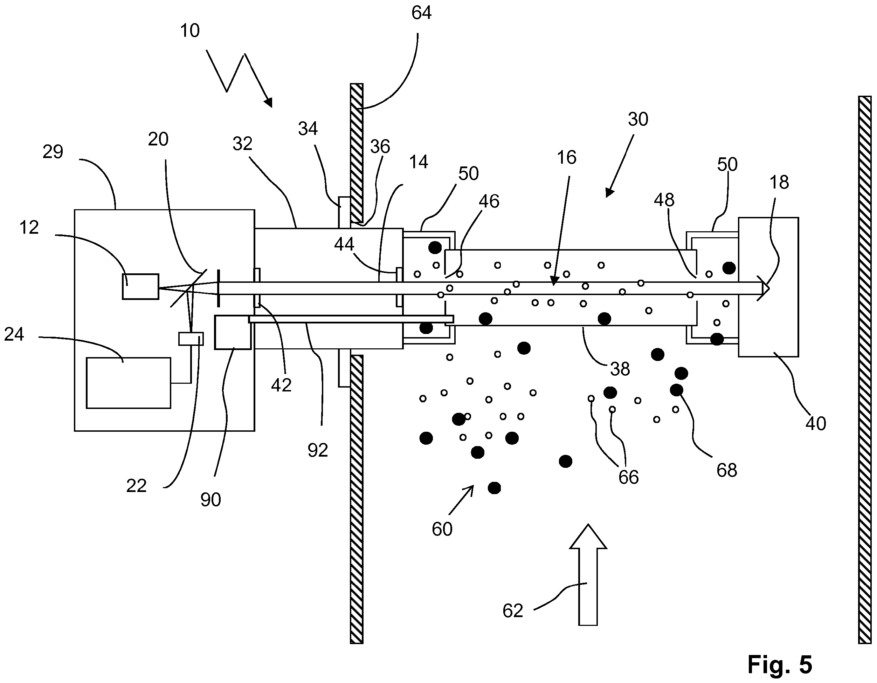

In einer vierten Ausführungsform Fig. 5 weist der Gasanalysator 10 eine Spülluftvorrichtung 90 auf, mit der ein Druckluftstoss über eine Rohrleitung 92 in und durch das Mantelrohr 38 blasbar ist, um das Mantelrohr 38 von Zeit zu Zeit, z.B. im 2-Stundentakt, von sich darin abgelagerten Partikeln 68 zu befreien.

Prinzipiell ist die Erfindung auch in sogenannten "cross-duct" Anwendungen einsetzbar. In solchen Anwendungen ist der Gasanalysator zweigeteilt aufgebaut und weist einen ersten Vorrichtungsteil auf, der wie das Gehäuse 29 mit seinen elektro-optischen Komponenten des ersten Ausführungsbeispiels aufgebaut ist und einen zweiten Vorrichtungsteil, der auf der gegenüberliegenden Seite des Kamins 64 angeordnet ist und in dem beispielsweise der Reflektor angeordnet sein könnte. In diesem zweiten Vorrichtungsteil kann auch ein zweiter Lichtempfänger angeordnet sein, der so angeordnet ist, dass er beispielsweise Streulicht empfangen kann, so dass mit dem Gasanalysator dann auch nach dem Prinzip der Streulichtmessung eine Konzentrationsauswertung von Gasanteilen vorgenommen werden kann. Das mit dem zweiten Lichtempfänger aufgenommene Streulicht kann dazu in einer zweiten Auswerteeinrichtung ausgewertet werden.

## Patentansprüche

1. Gasanalysator zur optischen In-situ Gasanalyse in einem Gasstrom (60), der neben einem zu messenden Gasanteil, dessen Konzentration bestimmt werden soll, die Konzentrationsbestimmung störende Partikel (68), wie Staub, Rauch, Wassertröpfchen oder sonstige Aerosole, enthält, mit
- einem Lichtsender (12) und einem Lichtempfänger (22), die zusammen eine optische Messstrecke in einem Messvolumen (16) definieren,
- einer Auswerteeinrichtung (24) zur Auswertung von Empfangssignalen des Lichtempfängers (22) zur Konzentrationsbestimmung,
- einer Ablenkvorrichtung zur Ablenkung der störenden Partikel (68), um diese Partikel (68) am Messvolumen (16) vorbei strömen zu lassen,
- wobei die Ablenkvorrichtung als quer zur Gasströmung (60) liegendes Mantelrohr (38) ausgebildet ist, in dessen Innerem das Messvolumen (16) liegt,
- **dadurch gekennzeichnet, dass** das Mantelrohr (38) einen geschlossenen Mantel aufweist und die Stirnseiten Öffnungen (46 und 48) zum Durchlassen von Gas (60) und Licht (14) des Lichtsenders (12) in das Messvolumen (16) aufweisen.

2. Gasanalysator nach Anspruch 1, **dadurch gekennzeichnet, dass** die Stirnseiten Leitbleche aufweisen.

3. Gasanalysator nach Anspruch 2, **dadurch gekennzeichnet, dass** die Leitbleche als flacher Deckel der Stirnseiten ausgebildet sind und die Öffnungen aufweisen.

4. Gasanalysator nach einem der vorhergehenden Ansprüche, **dadurch gekennzeichnet, dass** die Öffnungen in den Stirnseiten kleiner sind als der Querschnitt des Mantelrohres im Bereich des Messvolumens.

5. Gasanalysator nach einem der vorhergehenden Ansprüche, **dadurch gekennzeichnet, dass** jede der Öffnungen der Stirnseiten einen Ablenkring aufweist.

6. Gasanalysator nach einem der vorhergehenden Ansprüche, **dadurch gekennzeichnet, dass** eine Spülluftvorrichtung vorgesehen ist, mit der ein Druckluftstoss durch das Mantelrohr blasbar ist.

## Claims

1. Gas analyzer for optical in-situ gas analysis in a gas stream (60) containing, in addition to a gas fraction to be measured whose concentration is to be determined, particles (68) which interfere with the determination of the concentration, such as dust, smoke, water droplets or other aerosols, comprising
- a light transmitter (12) and a light receiver (22), which together define an optical measurement path in a measurement volume (16),
- an evaluation device (24) for evaluating received signals of the light receiver (22) for determining the concentration,
- a deflection device for deflecting the interfering particles (68) in order to cause these particles (68) to flow past the measuring volume (16),
- wherein the deflection device is in the form of a jacket tube (38) which lies transversely to the gas flow (60) and inside which the measuring volume (16) lies,
**characterised in that**
- the jacket tube (38) has a closed jacket and the end faces have openings (46 and 48) for the passage of gas (60) and light (14) from the light transmitter (12) into the measuring volume (16).

2. Gas analyser according to claim 1, **characterised in that** the end faces have guide plates.

3. Gas analyser according to claim 2, **characterised in that** the guide plates are constructed as a flat cover of the end faces and have openings.

4. Gas analyser according to one of the preceding claims, **characterised in that** the openings in the end faces are smaller than the cross-section of the casing pipe in the region of the measuring volume.

5. Gas analyser according to one of the preceding claims, **characterised in that** each of the openings of the end faces has a deflection ring.

6. Gas analyser according to one of the preceding claims, **characterised in that** a purge air device is provided with which a compressed air blast can be blown through the casing pipe.

## Revendications

1. Analyseur de gaz pour l'analyse optique in situ de gaz dans un courant gazeux (60) contenant, en plus d'une fraction de gaz à mesurer dont la concentration est à déterminer, des particules (68) qui interfèrent avec la détermination de la concentration, telles que poussière, fumée, gouttelettes d'eau ou autres aérosols, comprenant
- un émetteur de lumière (12) et un récepteur de lumière (22), qui définissent ensemble un trajet de mesure optique dans un volume de mesure (16),
- un dispositif d'évaluation (24) pour évaluer les signaux reçus du récepteur de lumière (22) pour déterminer la concentration,
- un dispositif de déviation pour dévier les particules interférentes (68) afin de faire passer ces particules (68) devant le volume de mesure (16),
- dans lequel le dispositif de déviation est sous la forme d'un tube d'enveloppe (38) qui se trouve transversalement à l'écoulement de gaz (60) et à l'intérieur duquel se trouve le volume de mesure (16),
**caractérisé en ce que**
- le tube d'enveloppe (38) présente une enveloppe fermée et les faces d'extrémité présentent des ouvertures (46 et 48) pour le passage de gaz (60) et de lumière (14) de l'émetteur de lumière (12) dans le volume de mesure (16).

2. Analyseur de gaz selon la revendication 1, **caractérisé en ce que** les faces frontales présentent des plaques de guidage.

3. Analyseur de gaz selon la revendication 2, **caractérisé en ce que** les tôles de guidage sont réalisées sous la forme d'un couvercle plat des faces frontales et présentent des ouvertures.

4. Analyseur de gaz selon l'une des revendications précédentes, **caractérisé en ce que** les ouvertures dans les faces frontales sont plus petites que la section transversale du tube d'enveloppe dans la zone du volume de mesure.

5. Analyseur de gaz selon l'une des revendications précédentes, **caractérisé en ce que** chacune des ouvertures des faces frontales présente une bague de déviation.

6. Analyseur de gaz selon l'une des revendications précédentes, **caractérisé en ce qu'**un dispositif d'air de purge est prévu avec lequel un jet d'air comprimé peut être soufflé à travers le tube d'enveloppe.
